# EUROPEAN PATENT APPLICATION

(11) **EP 4 736 780 A1**
(43) Date of publication of application: **06.05.2026**
(21) Application number: 24209351.6
(22) Date of filing: 29.10.2024
(51) Int. Cl.: A61B 8/08, A61B 8/12, A61B 8/00, A61B 5/00, A61B 34/20, A61B 90/00

(54) **CATHETER DISTAL TIP FOR PERFORMING SENSOR ASSISTED NAVIGATION, CATHETER ASSEMBLY, AND ROBOTICALLY ASSISTED INTERVENTIONAL DEVICES**

(71) Applicant: Creganna Unlimited Company, Ballybrit, Galway H91 VN2T (IE); Tyco Electronics UK Ltd., Swindon, Wiltshire SN3 5HH (GB)
(72) Inventor: HART, Stephen, Galway, H91 VN2T (IE); MCDERMOTT, Bernard, Galway, H91 VN2T (IE); REGUS, Cristian S., Galway, H91 VN2T (IE); WISEMAN, Stephen, Swindon, SN3 5HH (GB); GOESSERINGER, Peter, Galway, H91 VN2T (IE); BETTS, Andrew, Galway, H91 VN2T (IE)
(74) Representative: Grünecker Patent- und Rechtsanwälte PartG mbB

(57) **Abstract**

The present disclosure relates to a steerable catheter distal tip for performing sensor assisted navigation, to a catheter assembly comprising a catheter distal capsule and tip, and to a robotically assisted interventional device. A steerable catheter distal tip for performing sensor assisted navigation comprises an ultrasonic transducer system (106) arranged to surround a circumference of the catheter distal tip (100), wherein the ultrasonic transducer system (106) is operable to generate distance signals indicative of distances between a plurality of sensing locations on the circumference of the catheter distal tip (106) from walls of a cavity in which the catheter distal tip (100) is located in operation; and a signal processing unit (112) comprising a controller, which is operable to cause the catheter distal tip (100) being steered along a defined navigation path based on the distance signals.

## Description

The present disclosure relates to a preferably steerable catheter distal tip for performing sensor assisted navigation, to a catheter assembly comprising a catheter distal capsule and tip, and to a robotically assisted interventional device.

Minimally invasive intravascular procedures allow the performance of therapeutic treatments of locations within a patient's body while requiring only relatively small access incisions. An intravascular procedure may, for example, eliminate the need for open-heart surgery, reducing risks, costs, and time associated with an open-heart procedure. The intravascular procedure also enables faster recovery times with lower associated costs and risks of complication. An example of an intravascular procedure that significantly reduces procedure and recovery time and cost over conventional surgery is a heart valve replacement or repair procedure in which an artificial valve or valve repair device is guided to the heart through the patient's vasculature. For example, a Transcatheter Mitral Valve repair (TMVr) procedure generally requires a catheter device to be inserted femorally and tracked through the vasculature to the right atrium of the heart, then navigated through a puncture in the intra-atrial septum to the left atrium. An aortic valve replacement device typically enters the femoral artery and traverses around the aortic arch to the location of the aortic valve.

The distal end of a transcatheter delivery device needs to be positioned precisely to ensure the optimal positioning of, for instance a prosthetic valve before deployment, valve repair devices, stents, electrophysiology devices or other diagnostic or therapeutic devices. Additionally, it is crucial that the inner walls of the vessels are not damaged by the advancing catheter distal tip. In particular, dislodged wall tissue or collagenous debris might cause arterial embolism.

The principles according to the present disclosure can be used advantageously for performing a Transcatheter Aortic Valve Replacement procedure (TAVR). During this minimally invasive procedure a bioprosthetic valve is inserted inside the defective native valve. The procedure may also be called a Transcatheter Aortic Valve Implantation (TAVI). Furthermore, the present disclosure is also applicable to various other minimally invasive procedures in which a catheter needs to be moved within a cavity, while minimizing the potential damage to boundaries of the cavity.

There exist different concepts for localizing parts of catheters in the body and to display the result to a physician.

However, there is still a need for providing means to optimally navigate the tip of a catheter through cavities in the body whilst minimizing contact with the vessel walls. For instance, the walls (or boundaries) of the cavity may be the walls of blood vessels through which the catheter distal tip has to move.

This object is solved by the subject matter of the independent claims. Advantageous examples of the present disclosure are the subject matter of several dependent claims.

The present disclosure is based on the idea that by providing a smart catheter distal tip with ultrasonic transducer-based navigation, contact of the distal end of the catheter device with the vessel walls can be minimized leading to less traumatic navigation of the catheter through the vessels. Signal processing electronics and a vector navigation algorithm can be used to center the catheter distal tip e.g. in a blood vessel during a medical procedure. One possible procedure is TAVR but the technique is extensible to additional structural heart, lung, et. al. procedures conducted via arterial pathway, or other bodily vessels, and any navigation of a catheter or instrument.

In particular, a catheter distal tip for performing sensor assisted navigation according to an aspect of the present disclosure comprises an ultrasonic transducer system, the ultrasonic transducer system being arranged to surround a circumference of the catheter distal tip, wherein the ultrasonic transducer system is operable to generate distance signals indicative of distances between a plurality of sensing locations on the circumference of the catheter distal tip from walls of a cavity in which the catheter distal tip is located. A signal processing unit is provided, comprising a controller, which is operable to cause the catheter distal tip being steered along a defined navigation path based on the distance signals. The catheter distal tip may be steerable or non-steerable.

With the catheter distal tip according to the present disclosure, the achieved clinical outcomes may be improved significantly. The distal end of typical devices can have tapers, steps and raised edges and can be larger in diameter than the following body of the device. This makes the distal end more prone to damage of the vessels whilst making contact. Any complications induced by vascular injury may be reduced by minimizing contact and particulate generation from the native valve leaflets, ascending aorta, or the aortic arch in the case of a TAVR procedure. Such an arrangement will also facilitate atraumatic crossing of a native valve annulus by enabling the tip and distal end to be centered in the annulus. This arrangement will also allow determination of the location of features such as coronary ostia to allow the physician to avoid obstruction of said ostia. Further, the overall procedure time can be decreased and the reliance on fluoroscopy as well as the radiation exposure in the operating room may be reduced.

Ultrasonic transducers have the advantage that they do not emit harmful radiation and thus have been well-established in medical practice for many decades.

According to an advantageous example, the ultrasonic transducer system is operable to generate the distance signals based on time-of-flight measurements. Ultrasonic sensors can measure distance and detect the presence of an object without making physical contact. They do so by producing and monitoring an ultrasonic echo. Depending on the sensor and object properties, the effective range in the anatomy is sub-millimeter to a few centimeters, in air it is between a few centimeters up to several meters. The ultrasonic sensor (or transducer) generates and emits ultrasonic pulses that are reflected back towards the sensor by an object that is within the field of view of the sensor.

The ultrasonic sensor comprises a piezoelectric transducer, which is able to convert an electrical signal into mechanical vibrations, and mechanical vibrations into an electrical signal. Therefore, the ultrasonic sensor may be a transceiver which operates as both a transmitter and receiver at a single frequency. The sensor is able to capture the difference in time between the emitted and received echo. Because the speed of sound in a particular medium is a known variable, the captured round-trip time can be used to calculate the distance between the sensor and object. This method of ultrasonic sensing is a time-of-flight (ToF) measurement based on the propagation time of sound. It has to be noted that the velocity of sound varies by the composition of the medium through which the sound propagates, as well as by temperature.

According to an advantageous example, the ultrasonic transducer system comprises a plurality of piezoelectric MEMS ultrasonic transducers, PMUT. The ultrasonic Time-of-Flight (ToF) sensors may also integrate a MEMS PMUT (Piezoelectric Micromachined Ultrasonic Transducer) with an ultra-low power SoC (system on chip) in a miniature reflowable package. Based on ultrasonic pulse-echo measurements, the ToF sensor provides accurate and robust range measurements with lowest possible required space.

For instance, the ultrasonic transducer system may comprise a plurality of panel-shaped arrays of sensor elements, the arrays being mounted on a carrier substrate encompassing the catheter distal tip. This geometry allows an almost complete 360° field of vision and furthermore can be assembled in a particular easy and economic manner on the catheter distal tip. The carrier substrate comprises for instance a flexible printed circuit board encircling the area of the catheter distal tip close behind its tip. Each of the panel-shaped arrays provides a different view of the 360° surroundings. For example, each panel-shaped array of sensor elements may have dimensions in the range between 1 mm x 1 mm and 5 mm x 5 mm, preferably about 2 mm x 2 mm, comprising e. g. 10 x 10 sensor elements (also referred to as cells).

According to an advantageous example, the carrier substrate has a polygonal cross-section across to a longitudinal axis of the catheter distal tip. Thereby it is ensured that each of the plurality of panel-shaped arrays of sensor elements is mounted butting against the surface and electrical contacts of the carrier substrate. This is advantageous from a mechanical standpoint. However, when applying small enough sensor elements, the carrier substrate may also have a rounded, e.g. a circular cross-section.

In order to detect further variables, e.g. for calibrating the measurement signals, additional sensors can be provided. According to advantageous examples, the catheter distal tip further comprises a temperature sensor and/or a force sensor and/or a pressure sensor and/or a sensor determining acoustic properties of a medium inside the cavity. These sensors may also communicate with the signal processing unit.

According to a further advantageous example, the catheter distal tip may comprise an acoustic beamforming unit which is associated with the ultrasonic transducer system to emit a plurality of ultrasonic beams from the sensing locations on the circumference of the catheter distal tip. In particular, when using a two-dimensional array of sensing elements, the beamforming unit may be operable to control the individual sensor elements in order to modify a signal amplitude and/or a time or phase delay characteristics and/or wherein the beamforming unit is operable to perform comparator based amplitude processing or analog/digital conversion.

Advantageously, the signal processing unit may be operable to calculate the navigation path as a centroid of calculated wall positions derived from the distance signals. The navigation path can be followed by performing corrective movements to counteract any deviation from the centroid. Advantageously, these corrective movements may be performed automatically while the catheter distal tip advances along its desired path. Alternatively or additionally, the signal processing unit may also alert the physician with proximity to obstructions.

The catheter distal tip according to a further example may comprise a memory for storing calculated wall positions derived from the distance signals and wherein the signal processing unit is operable to calculate a map of the cavity along the navigation path. Such a map may also be calculated by an external signal processor and can be stored for future use with the same patient or others as part of a database for future data analytics.

The present disclosure according to a second aspect relates to a catheter assembly for performing sensor assisted navigation, the catheter assembly comprising a catheter distal tip according to the previously explained aspects and examples. The catheter assembly may for instance comprise a catheter system usable for TAVR procedure. The technique according to the present disclosure improves minimally invasive cardiovascular procedures for aortic valve replacement. In particular, it improves maneuverability through the femoral artery and through the aortic arch over a guidewire, may improve the physician's situational awareness over in-situ imaging with fluoroscopy and/or radiography. Furthermore, the sensing technique according to the present disclosure will provide additional positional feedback in robotically assisted procedures where sense of touch is absent.

According to the present disclosure, the signal processing unit may be arranged within the steerable catheter distal tip or at a proximal end of the catheter assembly. If the signal processing unit is located close to the ultrasonic transducer system, this is advantageous because the signal driving the sensor elements and the sensor signals which have to be evaluated do not have to be transmitted a long distance along the catheter. Thus, disturbances can be kept at a minimum and the accuracy is improved. On the other hand, when arranging the signal processing unit outside the steerable catheter distal tip, the size and geometry of the signal processing unit are subject to much less limitations compared to a scenario where the signal processing unit is integrated within the catheter distal tip.

In order to allow for the catheter assembly to automatically navigate along a centroid within the cavity, in which the catheter is advancing, the controller may be operable to generate a corrective action navigation vector signal for causing the steerable catheter distal tip to correct its position with respect to the centroid of calculated wall positions.

According to a further advantageous example, the controller may be operable to use data from multiple surgical procedures and clinical outcomes, the data being stored and used as training data for signal processing to define an optimal navigation path in a current procedure. Thus, the safety and efficiency of the performed procedures may be further improved.

Finally, the present disclosure relates according to a further aspect to robotically assisted interventional procedure systems comprising a catheter assembly according to the aspects and examples of the present disclosure.

The accompanying drawings are incorporated into and form a part of the specification to illustrate several examples of the present disclosure. These drawings together with the description serve to explain the principles of the disclosure. The drawings are merely for the purpose of illustrating the preferred and alternative examples of how the disclosure can be made and used, and are not to be construed as limiting the disclosure to only the illustrated and described examples. Furthermore, several aspects of the examples may form-individually or in different combinations-solutions according to the present disclosure. Further features and advantages will become apparent from the following more particular description of the various examples of the disclosure, as illustrated in the accompanying drawings, in which like references refer to like elements, and wherein:
- **FIG. 1**: shows schematically a perspective view of a catheter distal tip according to the present disclosure;
- **FIG. 2**: shows schematically a front view of the catheter distal tip of Fig. 1;
- **FIG. 3**: shows a schematic perspective view of an ultrasonic transducer system;
- **FIG. 4**: shows a schematic unfurled layout of the ultrasonic transducer system;
- **FIG. 5**: shows a schematic depiction of the principle of a time-of-flight ultrasonic measurement;
- **FIG. 6**: shows a schematic perspective view of a catheter assembly in operation;
- **FIG. 7**: shows a measurement diagram for a first position of the catheter distal tip;
- **FIG. 8**: shows a measurement diagram for a second position of the catheter distal tip;
- **FIG. 9**: shows schematic representation of the blind spot length;
- **FIG. 10**: schematically illustrates the blind spot length as a function of the number of polygon sides and the radius;
- **FIG. 11**: shows a schematic unfurled layout of the ultrasonic transducer system according to a second example;
- **FIG. 12**: shows a schematic perspective view of a PMUT sensor element;
- **FIG. 13**: shows a further schematic perspective view of a PMUT sensor element;
- **FIG. 14**: shows a schematic sectional view of a PMUT sensor element;
- **FIG. 15**: shows a schematic of a sensor equipped TAVR device;
- **FIG. 16**: shows a detail of FIG. 15;
- **FIG. 17**: shows a schematic representation of the human heart;
- **FIG. 18A to E**: show a schematic illustration of the advancing of the catheter distal tip through a blood vessel.

The present disclosure will now be explained in more detail with reference to the Figures.

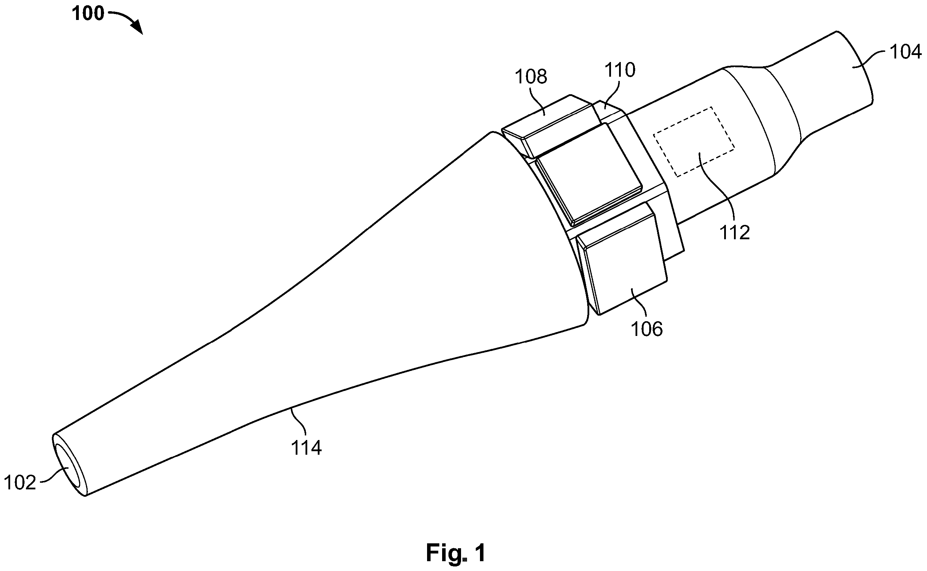

Fig. 1 shows a schematic perspective representation of a steerable catheter distal tip 100 according to the present disclosure. The catheter distal tip 100 has a distal end 102 and a proximal end 104. The proximal end 104 can be attached to the catheter body containing the steering section, so that the catheter distal tip 100 forms the tip (a distal end) of a catheter assembly. As used herein, the terms "proximal" and "distal" are to be taken as relative to a user using the disclosed delivery devices. "Proximal" is to be understood as relatively closer to the user and "distal" is to be understood as relatively farther away from the user.

Referring to Fig. 1, the distal tip 100, according to the present disclosure, comprises an ultrasonic transducer system 106. The ultrasonic transducer system 106 comprises exemplarily six planar arrays 108 of sensor elements, which will be discussed in more detail below. The arrays 108 are mounted on a carrier substrate 110. The carrier substrate is, for instance, formed from a flexible printed circuit board and is bent to have an annular shape. The carrier substrate 110 comprises means for electrically contacting the transducer arrays 108, as well as electrical leads that allow the connection to further electronic components, such as a signal processing unit 112 with a controller, and a power supply.

The signal processing unit 112 is schematically depicted as being located within the housing 114 of the distal tip 100. However, it is clear that the signal processing unit 112 may also be located at another part of a catheter assembly having the catheter distal tip 100 at its distal end.

According to an aspect of the present disclosure, the ultrasonic transducer system 106 comprises arrays 108 of piezoelectric time-of-flight sensors. In particular, piezoelectric micromechanical ultrasonic transducers (PMUT) are envisaged by the present disclosure. The arrays 108 of PMUT devices are arranged on the flexible printed circuit board 110 in a manner that they encircle the circumference of the catheter distal tip 100, covering essentially the complete 360° of the catheter distal tip's surroundings. However, the arrays 108 of PMUT devices do not necessarily have to cover the complete circumference, they may also be arranged to monitor only a smaller section.

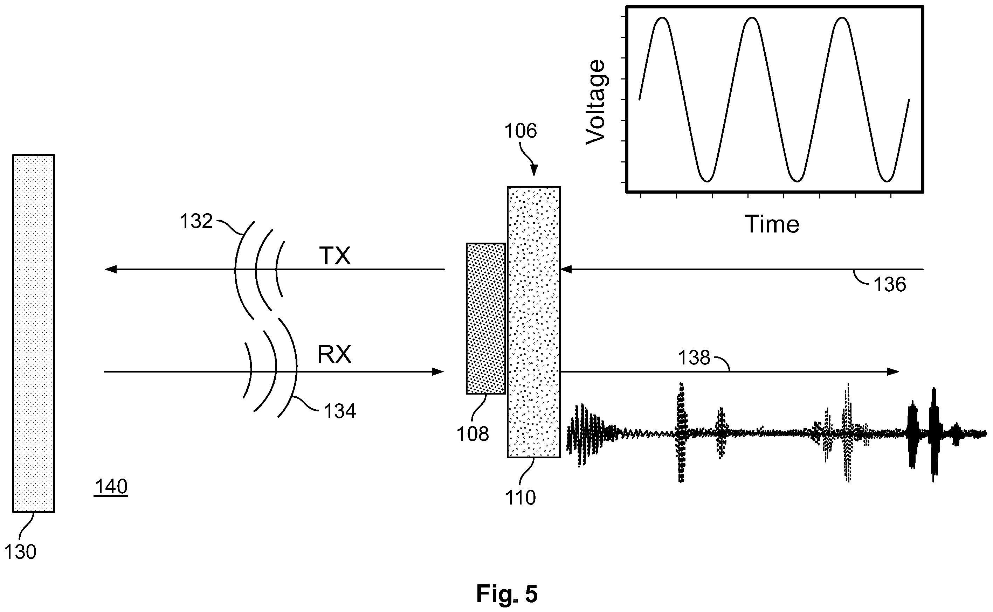

As shown in Fig. 1 and in more detail in Fig. 5, the ultrasonic transducer system 106 is operable to generate the distance signals based on time-of-flight measurements. The ultrasonic transducer system 106 comprises an array 108 of individual piezoelectric sensor elements, which generates and emits ultrasonic pulses that are reflected back towards the sensor by an object that is within the field of view of the sensor. The piezoelectric elements are able to convert an electrical signal into mechanical vibrations, and mechanical vibrations into an electrical signal. Therefore, the arrays 108 of the ultrasonic transducer system 106 each are transceivers which operate as both a transmitter and receiver at a desired frequency and bandwidth based upon the resonant modes activated by the drive electronics. Each array 108 is able to capture the difference in time between the emitted and received echo. Because the speed of sound in a particular medium (e.g. blood) is a known variable, the captured round-trip time can be used to calculate the distance between the array 108 of sensor elements and the object. This method of ultrasonic sensing is a time-of-flight (ToF) measurement based on the propagation time of sound. It has to be noted that the velocity of sound varies by the composition of the medium through which the sound propagates, as well as by temperature. Thus, additional sensors may be provided for measuring additional parameters (e.g. temperature, pressure etc.) for calibration and correction purposes.

For instance, the ultrasonic transducer system 106 may comprise a plurality of panel-shaped arrays 108 of sensor elements, the arrays 108 being mounted on the carrier substrate 110 encompassing the steerable catheter distal tip 100. This geometry allows an almost complete 360° field of view and furthermore can be assembled in a particular easy and economic manner on the distal tip 100. The carrier substrate 110 comprises for instance a flexible printed circuit board encircling the area of the catheter distal tip 100 close behind its tip. Each of the panel-shaped arrays 108 provides a different view of the 360° surroundings. For example, each panel-shaped array 108 of sensor elements may have dimensions in the range between 1 mm x 1mm and 5 mm x 5 mm, preferably about 2 mm x 2 mm, comprising e. g. 10 x 10 piezoelectric sensor elements (also referred to as cells).

Although not shown in the Figures, the ultrasonic transducer system 106 may additionally comprise a protective outer sheath which protects the components from the surrounding medium.

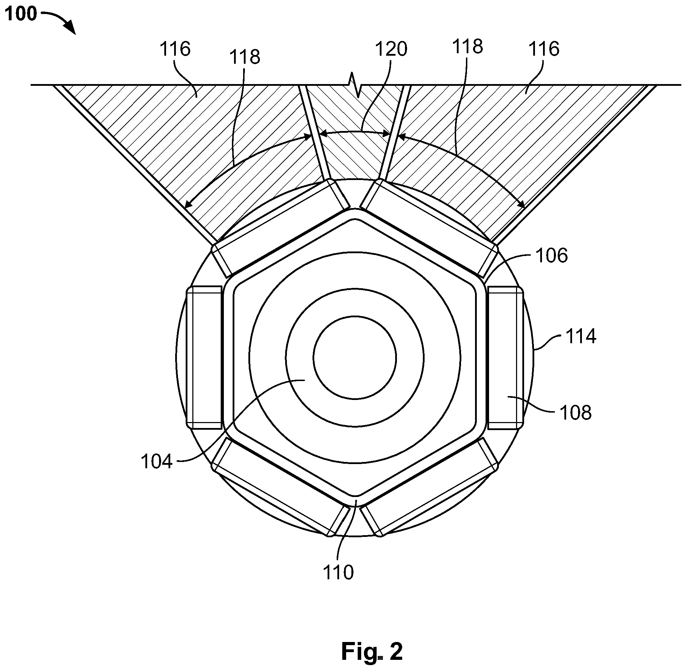

Fig. 2 shows a rotated view from the proximal end 104 onto the distal tip 100 of Fig. 1.

As illustrated schematically for two of ultrasonic sensor elements, each of the arrays 108 emits a transmission beam 116. Further, also the reflected ultrasonic beam can be detected within the beam angle 118. Between these illuminated regions 116, there may remain a blind angle or blind spot 120. The size of the blind spot 120 can be reduced by enhancing the number of arrays 108.

Ideally, the piezoelectric sensor elements are distributed evenly around a circular carrier substrate 110. For facilitating the fabrication, however, using a hexagonal cross-section for the ultrasonic transducer system 106 has been proven to yield satisfactory results.

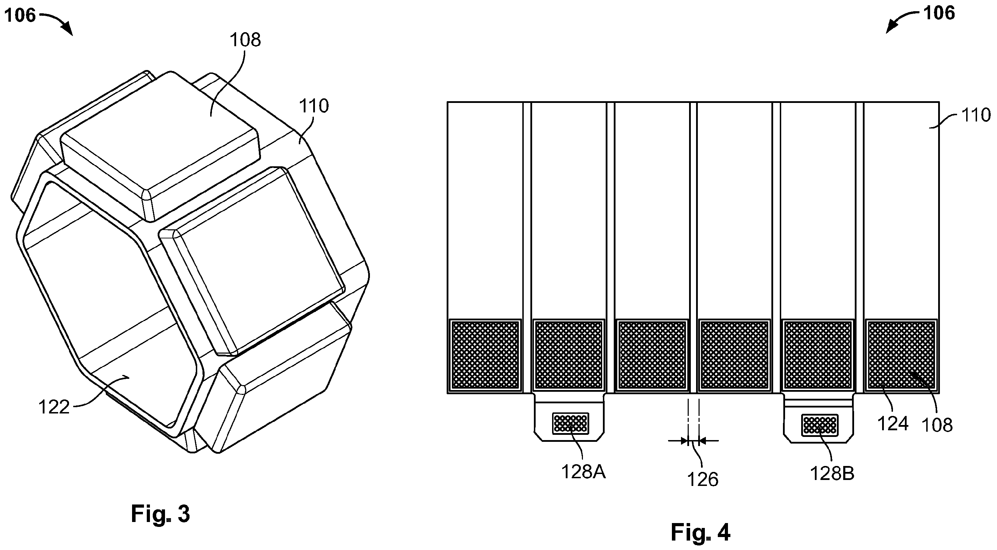

Fig. 3 illustrates a perspective view of the ultrasonic transducer system 106 using six arrays 108 of sensor elements. The arrays 108 are arranged on the carrier substrate 110 and electrically connected to electrical leads within the carrier substrate 110, not visible in the Figure. On the inner surface of the ultrasonic transducer system 106, an adhesive layer 122 is arranged, which allows fixing the ultrasonic transducer system 106 on the housing 114 of the catheter distal tip 100, as shown in Figures 1 and 2.

However, the ultrasonic transducer system 106 may, of course, also be attached to the housing 114 of the catheter distal tip 100 by form-fit, overmolding, snap-fit connections, and/or ultrasonic welding.

Figure 4 shows the layout of the unfurled ultrasonic transducer system 106. According to this particular example, six arrays 108 are provided and mounted on a flexible printed circuit board 110. The flexible printed circuit board may, for instance, be formed by a flexible substrate comprising biaxially-oriented polyethylene terephthalate (BoPET) or polyimide (PI). Each of the arrays 108 comprises a plurality of individual sensor elements 124. In the shown embodiment, each array 108 comprises a 10 x 10 matrix of piezoelectric sensor elements 124.

As explained with reference to Fig. 2, the arrays 108 are arranged adjacent to each other but with a gap 126 between each other. This gap 126 is needed for folding the ultrasonic transducer system 106 into the tube-shaped structure with polygonal cross-section as shown in Fig. 3. The gap 126 may, for instance, have dimensions between 0.1 mm and 0.5 mm, preferably 0.3 mm.

Further, the ultrasonic transducer system 106 may also comprise additional sensors, such as temperature sensors or force sensors. Two of such sensors 128A, 128B are depicted in Fig. 4. They may be folded to the inside of the three-dimensional ultrasonic transducer system 106 shown in Fig. 3. Then the additional sensors 128A, 128B may for instance measure the temperature on the inside of the ultrasonic transducer system 106.

Fig. 5 shows a schematic simplified illustration of the ultrasonic transducer system 106 for explaining the principle of the ToF measurement according to the present disclosure in more detail.

As shown in Fig. 5, each of the arrays 108 arranged on the carrier substrate 110 is receiving an excitation voltage (or excitation signal) as indicated by the arrow 136. The piezoelectric sensor elements of the array 108 cause an acoustic wave 132 to be emitted from the array 108 into the surrounding medium 140. For the application of a TAVR procedure, the medium 140 is blood. When the emitted acoustic wave 132 impinges on a vessel, wall, or generally an obstruction 130, it is reflected. A reflected acoustic wave propagates back to the array 108 where it causes mechanical vibrations which then are converted into an electrical output signal 138.

As this is generally known in the art, the output signal 138 is subjected to a sophisticated signal processing in order to extract the time-of-flight information from the reflected acoustic waves.

It is known to drive transducers with a sine or square wave at their center frequency to achieve the best results. Most integrated solutions have an output driver consisting of low-side drivers to drive a transformer in a transformer-drive situation, or FETs in an H-bridge configuration for a direct drive solution. After the transducer sends out an echo at its resonant frequency, the system must then listen for return echoes which are a result of an object in the transducer's field of view. Ultrasonic systems typically filter the return echo to remove noise and gain the signal up before it goes to an ADC. Some ways to gain the ultrasonic system are as follows.

Using a digital gain applies a fixed gain to the entire ultrasonic echo. Further, using a time-varying-gain involves applying a gain that is dependent on how far out an object to be detected is. Often, objects that are further out in time produce a weaker echo response and objects that are closer produce a stronger echo response. To combat this, to prevent saturation of the close signals, and to be able to identify the further objects, one may choose to gain their system such that a small gain is applied early in time, and a larger gain is applied further out in time. This gives flexibility to configure the gain based on the system requirements. Finally, an automatic gain control by means of a logarithmic amplifier can be used. The logarithmic amplifier approach is a way to achieve automatic gain control when dealing with input signals that are both high and low in amplitude. The logarithmic amplifier gains an input signal based on the logarithmic scale which helps get a stronger echo response from weak signals while also appropriately gaining the strong signals but preventing saturation, similar to the time-varying-gain approach. Whereas the time-varying-gain method is dependent on where an object is in time, the logarithmic amplifier is dependent on the actual echo of the input signal itself with no dependency on time. Once the return signal has been filtered and gained appropriately, the data can be input to an analog-digital converter (ADC) for further signal processing.

After the signal is digitized, it may be further processed by a digital signal processor (DSP) or an MCU. In particular, the signal passes through a bandpass filter to reduce any out-of-band noise.

As symbolically depicted in Fig. 5, the output signal 138 comprises the measured signals from each of the individual arrays 108, in the exemplary case six output signals 138.

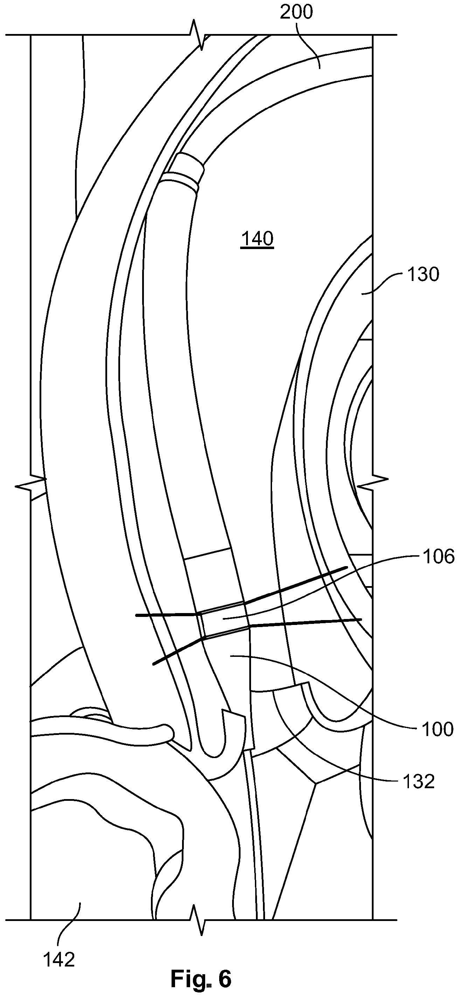

Fig. 6 depicts an exemplary application scenario where the distal tip 100 as part of a catheter assembly 200 has been introduced inside a blood vessel forming a cavity 142. However, all of the proximity sensing principles of the present disclosure may also be used in completely different application environments apart from the medical application field.

The ultrasonic transducer system 106 is operable to emit acoustic waves 132, which are reflected by the walls 130. Based on a ToF evaluation, a signal processing unit which may be located inside the catheter distal tip 100, calculates the distances between the center of the catheter distal tip 100 and the reflecting walls 130. Further, an approximation of the outline of the cavity boundaries is calculated.

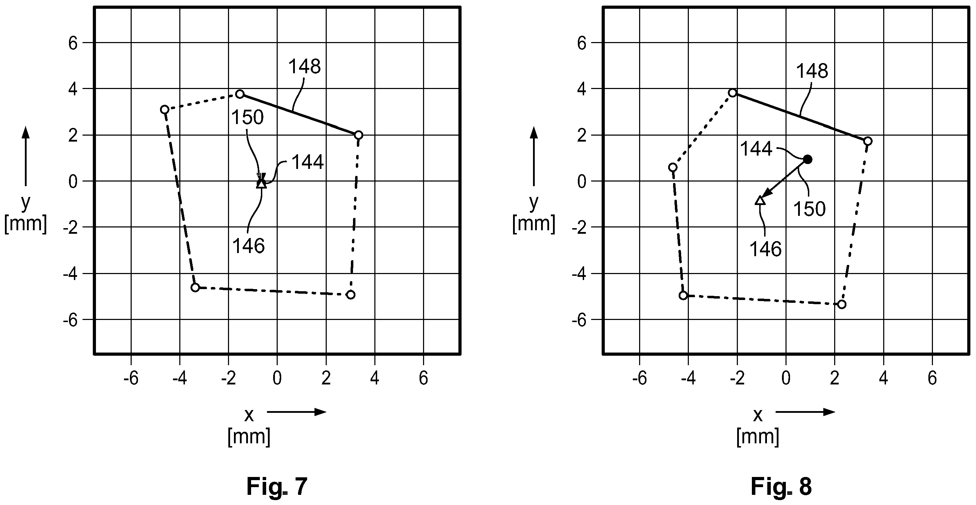

As illustrated in Fig. 7, from the approximated outline 148, a centroid 146 of the cavity 142 is calculated. Exemplary measurement results as shown in Figures 7 and 8 are obtained. In both diagrams, a cross-section of the space, through which the catheter distal tip is travelling, is shown. The coordinates x and y are given in mm.

Fig. 7 relates to the situation in which the position 144 of the catheter distal tip 100 is optimally aligned with a calculated midpoint 146 of the boundary 130 of the cavity 142. Consequently, the signal processing unit does not generate a signal causing the catheter distal tip to change its position in the x-y plane. This situation can for instance be mirrored by an output vector 150 indicating a command for deviating in the x-y plane being zero.

In contrast thereto, Fig. 8 shows the situation in which the position 144 of the catheter distal tip 100 is not aligned with a calculated midpoint 146 of the boundary 130 of the cavity 142. Consequently, the signal processing unit generates a signal causing the catheter distal tip 100 to change its position in the x-y plane. This situation can for instance be mirrored by a non-zero output vector 150 indicating a command for deviating in the x-y plane towards the midpoint 146.

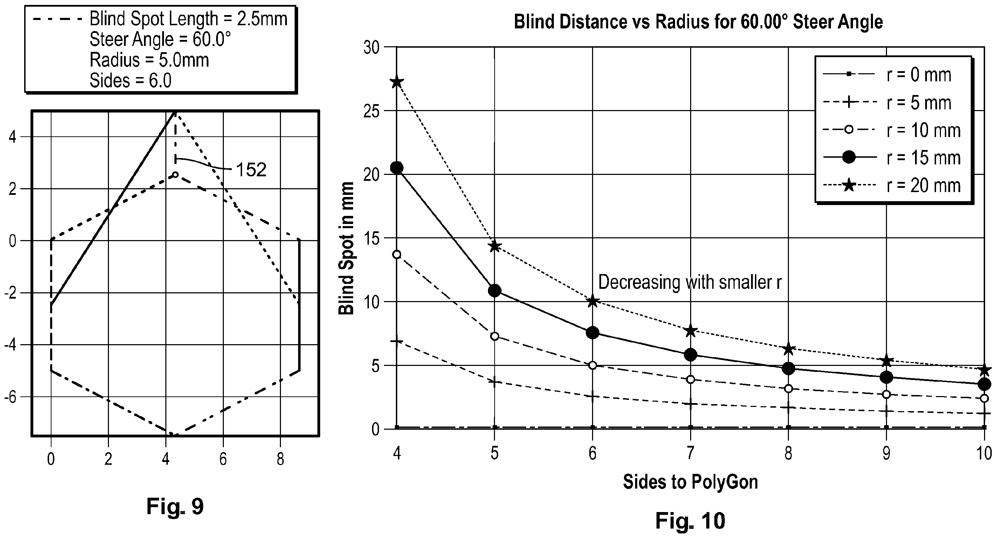

Depending on the blind angle 120 left by the arrays 108 of sensor elements as shown in Fig. 2, also a blind spot length 152 has to be considered. The blind spot length 152 is defined as a distance in the x-y plane in which an approach of the catheter distal tip 100 towards the boundary 130 cannot be detected. In Fig. 9 the blind spot length 152 is exemplarily shown for a hexagonal ultrasonic transducer system with six arrays 108 of sensor elements. The sensor elements 124 (see Fig. 4) are arranged in a 10x10 matrix. The individual sensor elements 124 can be grouped and addressed as 10 columns extending along the longitudinal axis of the catheter distal tip 100 (i.e. between the distal and the proximal end). If the exciting voltage applied to each column of sensor elements 124 is phase shifted with respect to the respectively adjacent column, the acoustic beams can be steered off-axis. In the example shown in Fig. 9, the length of the blind spot is dependent on the catheter distal tip radius and the off-axis steer angle. For instance, an angle of 60 degrees was chosen in Fig. 9.

Now referring to Fig. 10, the correlation between the blind spot length 152 and the number of arrays 108, in other words the number of sides to the polygon formed by the ultrasonic transducer system is shown. The radius r of the catheter distal tip 100 is given as a parameter of the set of curves, ranging from 0 to 9 mm. As can be seen from this Figure, enhancing the number of arrays from 4 to 6 leads to a rather significant improvement and reduction of the blind spot length. Enhancing the number from 6 to e. g. 8 has a much lesser effect and may not justify the additional complexity under certain cost and form factor restraints.

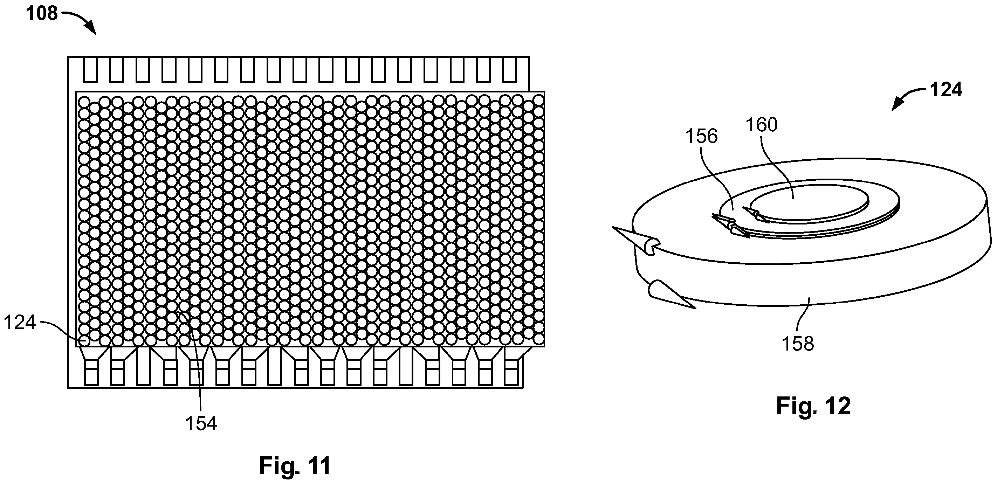

As already mentioned above, if the blind angle is to be reduced, the individual piezoelectric sensor elements 124, which may be grouped as sensor arrays 108, have to be distributed around the circumference of the catheter distal tip as evenly as possible. In the previously considered example (see Fig. 4), the sensor arrays 108 comprise a 10x10 matrix of the piezoelectric sensor elements 124. Fig. 11 shows a different layout of the array 108 of sensor elements 124.

Sensor elements 124 which are grouped to form a column 154 extending in a longitudinal direction can be addressed to form a channel. In other words, each single PMUT forms an element, a row of N PMUTs forms a linear array, and N rows of linear arrays form a matrix array.

The construction and functionality of the individual piezoelectric sensor elements 124 will now be explained in more detail referring to Figures 12 to 14. However, the principles according to the present invention can also be applied to other ultrasonic transducers, e. g. based on capacitive-based transducers (CMUTs), bulk piezoelectric transducers, or polyvinylidenfluoride (PVDF) films.

Compared with bulk ultrasonic transducers, the micro-machined ultrasonic transducers (MUTs) are easy to fabricate even for high acoustic frequencies (over 10 MHz), can be produced in large arrays with a small footprint, and mostly have high process compatibility with standard integrated circuit production. Therefore, MUTs are promising elements for high-frequency 2D phased arrays. MUTs are divided into capacitive-based transducers (CMUTs) and piezoelectric-based transducers (PMUTs). Compared to CMUT, PMUTs have a more simple structure and thus a robust fabrication process, generate higher acoustic beam intensity, and do not require any bias voltage, which is preferred in medical applications.

Thus, the present disclosure in the following focuses on PMUT arrays as the sensing technology.

As shown in Fig. 12, one PMUT sensor element 124 comprises a piezoelectric deformable layer 156 comprising e.g. lead zirconate titanate (PZT), barium titanate, lead titanate, or aluminium nitride (AIN). The piezoelectric deformable layer 156 is mounted on a support structure (a substrate) 158. On the substrate 158 there is a bottom electrode 162 which contacts the piezoelectric layer 156 from the lower side in Figure 12. For contacting the piezoelectric layer 156 from the opposing side, a top electrode 160 is provided.

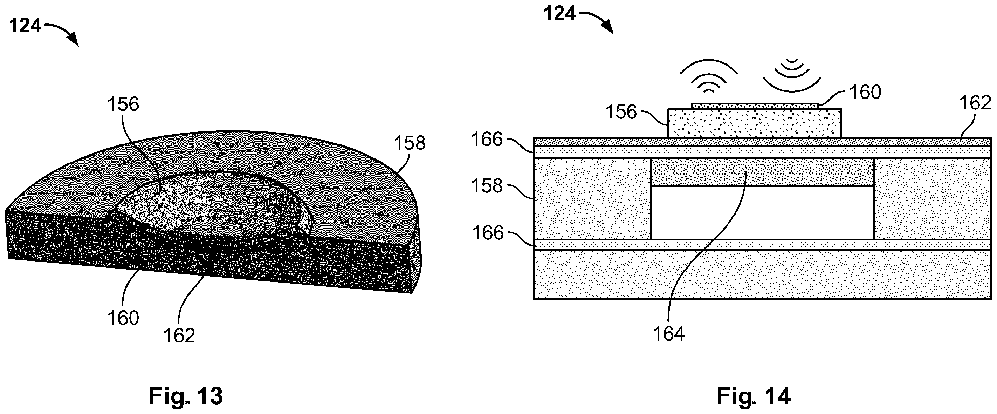

The PMUT 124 is capable of generating and detecting acoustic waves via the piezoelectric deformable layer 156 between first and second electrodes embracing the structural thin film or films forming a suspended piezoelectric layer or membrane. The top electrode 160 is used to operably transmit the ultrasonic wave via dynamic excitation of the piezoelectric layer by an electrical signal. Each individual PMUT of the array 108 is addressable through phase shifted signals respectively applied to the top electrode portions. The piezoelectric layer 156 can be clamped or otherwise operably disposed between the electrodes 160 for excitation by application of electric signals to the top electrode portions, and it may be circular, rectangular, square, or any other two-dimensional shape (see Fig. 14).

Fig. 14 demonstrates a cross-sectional schematic of a typical PMUT 124. In the cross-sectional view, a first or top electrode 160 is situated at the top of the PMUT 124, and it may be placed directly on top of a piezoelectric layer 156, which separates the top electrode 160 and a second or bottom electrode 162. It is noted that designations such as top, bottom, above, below and the like are used throughout to describe orientation of structures as depicted in the drawings, and should not be taken to indicate a required position or orientation of structures in actual implementations of devices in accordance with the herein described embodiments.

Below the second electrode 162 is a structural layer 164, the thickness of the structural layer 164 allows for adjustment of the tuning frequency of the piezoelectric layer 156 and hence the operation of the PMUT, through conventional piezoelectric design principles. Isolation dielectric layers 166 comprise e.g. SiOx and a support structure 158 may be made of, e.g., silicon.

The piezoelectric layer 156 is formed as a thin layer or membrane, which is fabricated using known techniques of microfabrication from a suitable material, such as aluminum nitride (AIN), although other conventional piezoelectric materials may be used. As such, the suspended piezoelectric layer 156 can mechanically deform and oscillate if a time-varying electrical signal is applied to the first and second electrodes 160 and 162. Reciprocally, if an oscillating mechanical force, such as an acoustic pressure caused by a reflected ultrasonic pulse, is applied to the piezoelectric layer 156, it will vibrate and in turn produce a time-varying electrical signal that will be detected at the electrodes 160 and 162.

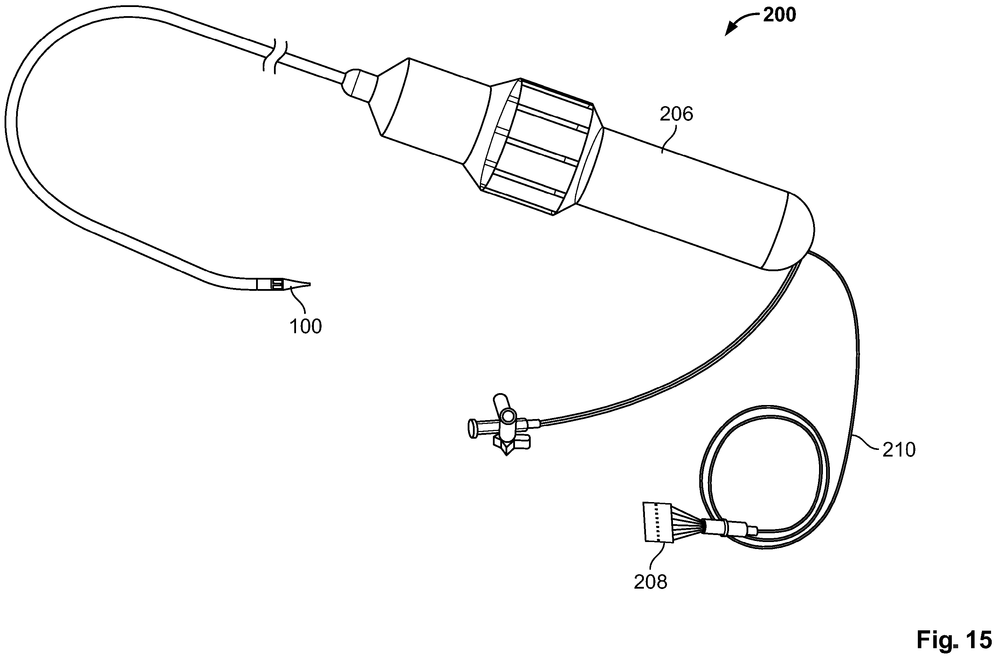

Figure 15 illustrates a catheter assembly 200 in which the above described distal tip 100 can advantageously be used.

The catheter assembly 200 is a configuration suitable for performing a self expanding TAVR procedure. To this end a bioprosthesis is compressed into a retractable sheath proximal of the distal tip 100 containing the sensors. A handle 206 allows the physician to advance the catheter through the vessels to the therapy site in a longitudinal direction. According to one embodiment of the principles of the present disclosure, the catheter assembly 200 is steerable at the distal end to allow the physician or a robotic system to adjust the location of the distal tip of the catheter relative to the vessel walls or features of the anatomy by using the output signals generated by the ultrasonic transducer system 106.

Thereby the distal tip 100 can be steered away from any obstacles and in particular from the boundaries of the cavity through which the catheter distal tip 100 is traveling.

The distal tip and therefore the unexpanded prosthetic valve can be positioned optimally in the center of the native valve annulus before deployment.

The location of the coronary ostia relative to the implant can be verified before deployment thereby facilitating optimal commissure alignment.

An electric connector 208 and an electrical lead 210 may be provided to connect the catheter assembly 200 to external components, in particular a power supply. Of course, also an interface unit for a wireless transmission may also be provided.

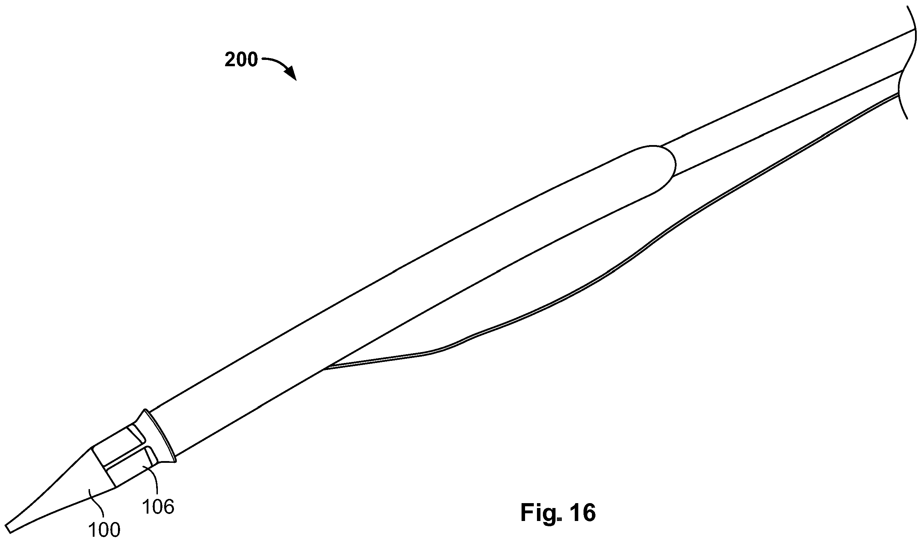

Figure 16 illustrates a detail of the catheter assembly 200, in particular the assembled catheter distal tip 100 with the ultrasonic transducer system 106.

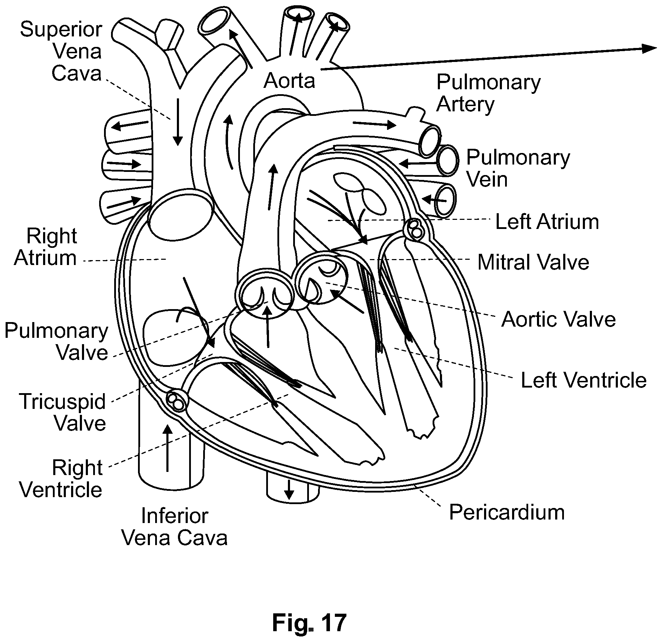

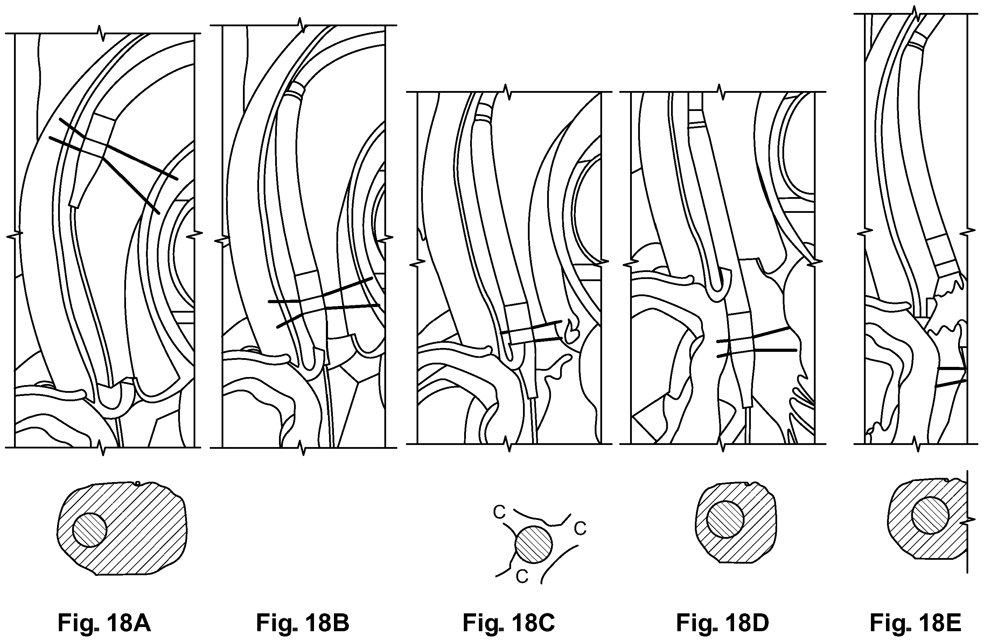

With reference to Figures 17 and 18, the steering procedure in an actual application environment is illustrated in more detail. Fig. 17 Illustrates the main components of a human heart.

Figures 18A to 18E illustrate the progression of the catheter distal tip 100 towards the deploy position during a TAVR procedure. The catheter in this application has to pass through a relatively small diameter bend with optimally minimal contact of the catheter tip to the vessel walls and native valve leaflets to reduce the risk of generation of embolic debris and risk of tissue damage. It is also optimal to pass centrally through the native annulus, avoiding the native valve leaflets and remain central during the prosthesis deployment. Further, it is also advantageous to be able to locate features such as the coronary ostia. Using proximity sensing can facilitate this optimal placement and deployment.

As shown in Figure 18A, the tip is positioned relative to the ascending aorta and can be steered to be centralized.

Fig. 18B shows the position of the tip relative to the ascending aorta, when close to the native valve. The tip may be steered to be centralized before entering the native valve. The tip may also be able to identify the coronary ostia to allow commissure alignment.

In the next step, as shown in Fig. 18C, the position of the tip are determined relative to the leaflets and can be centralized with respect to the leaflets. It should be noted that in this position the leaflets will be moving into and out of contact. Advantageously, the position of the commissures C can also be determined.

Fig. 18D depicts the position of the tip relative to the left ventricular outflow tract (LVOT), also sometimes called the aortic vestibule, prior to deployment of the artificial valve. The tip can be steered to be centralized.

Finally, Fig. 18E illustrates the position of the tip during deploying the artificial valve. In this position, the tip can be adjusted to center the capsule during deployment.

Thus, the catheter distal tip 100 and catheter assembly 200 according to the present disclosure reduce the risk of generation of embolic debris and damage to tissues and may assist in optimal placement and deployment of prosthetic valve. TAVR is one application and this concept is applicable to all therapies to enable atraumatic navigation through vessels and to aid placement and deployment of many implantable devices, to aid positioning of repair devices, electrophysiology (EP) devices and other devices where accurate location at the distal tip is required.

In summary, the present disclosure provides an improved catheter navigation technology concept addressing the growing opportunity for minimally invasive cardiac procedures like transcatheter aortic valve replacement (TAVR). These devices may improve TAVR procedures by improving clinical outcomes through reduced catheter interaction with the aortic arch inner walls. For TAVR and other minimally invasive procedures, improving physician awareness of contact forces and the relative position at the distal end of such catheter devices enables the physician to navigate more judiciously within the anatomy, minimizing vascular harm. This can reduce vascular injury related complications thereby reducing patient recovery times and overall post-operative mortality. It also reduces reliance on fluoroscopy, procedure time, and radiation exposure of all parties. A risk of post-procedural complications comes from dislodged debris, particularly occurring in the aortic valve, ascending aorta, or the aortic arch causing an embolism in the supra-aortic arteries.

Piezoelectric Micromachined Ultrasound Transducer (PMUT) technology may be used as an aide for catheter steering capabilities for Transcatheter aortic valve replacement (TAVR) or neurological applications, at/or below the millimeter range. A PMUT array placed on a catheter's tip enables proximity detection of surrounding artery walls and obstructions utilizing ultrasound and preventing the actual contact/pressure of the catheter distal tip against the artery walls. The navigation algorithm and electronics methodology enables an improved steering of the catheter distal tip. They may also allow identification of features such as coronary ostia during procedures.

Such proximity sensing capabilities will enable or enhance manual, robotically assisted or robotic navigation of devices through the anatomy by identifying correct or optimal pathways through the anatomy, allowing precise, atraumatic navigation, for instance through tortuous arteries in the brain or heart.

Other variations and/or modifications and/or enhancements include other structural heart procedure, any robotic surgery, surgical application enhancements to optimize navigation during procedures (e.g. fast, slow, contact force limits), vector navigation added to surgical screen or monitor during procedure or the addition of haptic feedback.

This disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

It should also be noted that proximity sensing is applicable to non-steering devices as well as steerable devices.

Proximity sensing can be used for physician training purposed where an experienced practitioner can demonstrate expert procedure technique to less experienced practitioners with the additional proximity sensing images supplementing and clarifying the radioscopy images.

Proximity sensing may assist in identifying anatomical features to aid real time registration of anatomical models with live anatomy.

Furthermore, proximity sensing may also be useful in other application environments outside the medical field, e.g. in robotics, obstacle detection and avoidance systems, industrial automation, parking sensors, etc.

It should also be noted that the catheter distal tip according to the present disclosure may also comprise a wireless communication interface for wirelessly transmitting and/or receiving signals.

Furthermore, the catheter tips according to the present disclosure can be realized with a low resolution and/or a low cost-size to allow these to be used on single use devices.

**REFERENCE NUMERALS**

| **Reference Numeral** | **Description** |
|---|---|
| 100 | Catheter distal tip |
| 102 | distal end |
| 104 | proximal end |
| 106 | ultrasonic transducer system |
| 108 | array of sensor elements |
| 110 | carrier substrate |
| 112 | signal processing unit |
| 114 | housing of catheter distal tip |
| 116 | ultrasonic beam |
| 118 | beam angle |
| 120 | blind spot; blind angle |
| 122 | adhesive layer |
| 124 | piezoelectric sensor element |
| 126 | gap |
| 128A, 128B | additional sensor |
| 130 | boundary of cavity |
| 132 | emitted acoustic wave |
| 134 | reflected acoustic wave |
| 136 | excitation voltage |
| 138 | output signal |
| 140 | surrounding medium; blood |
| 142 | cavity |
| 144 | calculated position of the catheter distal tip |
| 146 | calculated centroid of the cavity |
| 148 | approximated outline of cavity |
| 150 | output vector |
| 152 | blind spot length |
| 154 | column of sensor elements forming a channel |
| 156 | piezoelectric deformable layer |
| 158 | support structure |
| 160 | top electrode |
| 162 | bottom electrode |
| 164 | structural layer |
| 166 | isolation dielectric layer |
| 200 | catheter assembly |
| 206 | handle |
| 208 | electrical connector |
| 210 | electrical lead |

## Claims

1. Catheter distal tip for performing sensor assisted navigation, the catheter distal tip (100) comprising:
an ultrasonic transducer system (106) arranged to surround a circumference of the catheter distal tip (100), wherein the ultrasonic transducer system (106) is operable to generate distance signals indicative of distances between a plurality of sensing locations on the circumference of the catheter distal tip (106) from walls of a cavity in which the catheter distal tip (100) is located in operation;
a signal processing unit (112) comprising a controller, which is operable to cause the catheter distal tip (100) being steered along a defined navigation path based on the distance signals.

2. Catheter distal tip according to claim 1, wherein the ultrasonic transducer system (106) is operable to generate the distance signals based on time-of-flight measurements.

3. Catheter distal tip according to claim 1 or 2, wherein the ultrasonic transducer system (106) comprises a plurality of piezoelectric MEMS ultrasonic transducers, PMUT.

4. Catheter distal tip according to one of the preceding claims, wherein the ultrasonic transducer system (106) comprises a plurality of panel-shaped arrays (108) of sensor elements (124), the arrays (108) being mounted on a carrier substrate (110) encompassing the steerable catheter distal tip (100).

5. Catheter distal tip according to claim 4, wherein the carrier substrate (110) has a polygonal cross-section across to a longitudinal axis of the steerable catheter distal tip (100).

6. Catheter distal tip according to one of the preceding claims, further comprising a temperature sensor and/or a force sensor and/or a pressure sensor and/or a sensor determining acoustic properties of a medium inside the cavity (142).

7. Catheter distal tip according to one of the preceding claims, further comprising an acoustic beamforming unit which is associated with the ultrasonic transducer system (106) to emit a plurality of ultrasonic beams from the sensing locations on the circumference of the catheter distal tip (100).

8. Catheter distal tip according to claim 7, wherein the beamforming unit is operable to modify a signal amplitude and/or a time or phase delay characteristics and/or wherein the beamforming unit is operable to perform comparator based amplitude processing and/or analog/digital conversion.

9. Catheter distal tip according to one of the preceding claims, wherein the signal processing unit (112) is operable to calculate the navigation path as a centroid of calculated wall positions derived from the distance signals.

10. Catheter distal tip according to one of the preceding claims, further comprising a memory for storing calculated wall positions derived from the distance signals and wherein the signal processing unit (112) is operable to calculate a map of the cavity along the navigation path.

11. Catheter assembly for performing sensor assisted navigation, the catheter assembly (200) comprising a catheter distal tip (100) according to the preceding claims.

12. Catheter assembly according to claim 11, wherein the signal processing unit (112) is arranged within the steerable catheter distal tip (100) or wherein the signal processing unit (112) is arranged at a proximal end of the catheter assembly (200).

13. Catheter assembly according to claim 11 or 12, wherein the controller is operable to generate a corrective action navigation vector signal for causing the steerable catheter distal tip (100) to correct its position with respect to a centroid of calculated wall positions.

14. Catheter assembly according to one of the claims 11 to 13, wherein the controller is operable to use data from multiple surgical procedures and clinical outcomes, the data being stored and used as training data for signal processing to define an optimal navigation path in a current procedure.

15. Interventional robotic system comprising a catheter assembly (200) according to one of the claims 11 to 14.

16. Interventional robotic system according to claim 15, comprising further sensor elements arranged on an outer surface of a catheter.
